# EUROPEAN PATENT APPLICATION

(11) **EP 1 911 489 A1**
(43) Date of publication of application: **16.04.2008**
(21) Application number: 06782426.8
(22) Date of filing: 07.08.2006
(51) Int. Cl.: A61N 1/30

(54) **IONTOPHORESIS DEVICE**

(30) Priority: 05.08.2005 JP 2005228529
(71) Applicant: TTI Ellebeau, Inc., Tokyo 150-0022 (JP)
(72) Inventor: NAKAYAMA, Mizuo, TTI ellebeau, Inc., Tokyo 1500022 (JP); MATSUMURA, Takehiko, c/o TTI ellebeau, Inc., Tokyo 150-0022 (JP); AKIYAMA, Hidero, c/o TTI ellebeau, Inc., Tokyo 1500022 (JP); MATSUMURA, Akihiko, TTI ellebeau, Inc., Tokyo 1500022 (JP)
(74) Representative: Wagner, Karl H.
(86) International application number: PCT/JP2006/315584
(87) International publication number: WO 2007/018171

(57) **Abstract**

A patch-type iontophoresis device (10) comprising a working electrode assembly (12), a non-working electrode assembly, a DC electric power source 16 and an electrically conductive cord 62 for connecting the working electrode assembly 12 and the non-working electrode assembly are arranged on a coupling belt 54 for coupling the working electrode assembly 12 and the non-working electrode assembly 14, a current value setting device 70 being provided with an electrical resistor 72 in parallel with the electrically conductive cord 62, a connector 74 being stuck to arbitrary portions of the electrical resistor 72 and the electrically conductive cord 62 upon setting of a DC value so that the resistor and the cord are connected to each other, and an insulating film 75Abeing provided with the connector 74 stuck to the coupling belt 54 with the aid of an adhesive layer 75B.

## Description

### Technical Field

The present invention relates to an iontophoresis device for administering a drug ion to an organism.

### Background Art

Iontophoresis is one method of permeating a drug into an organism from the skin or mucosa of the organism. The iontophoresis includes a working electrode assembly having a drug solution holding portion holding a drug solution and a non-working electrode assembly as a counter electrode of the working electrode assembly. A voltage having the same conductivity type as that of a drug ion in the drug solution holding portion is applied to the working electrode assembly in a state where the drug solution is brought into contact with the skin or mucosa to electrically drive the drug ion so that the drug ion is transferred into an organism via the skin or mucosa of the organism.

One possible structure for each of the working electrode assembly and the non-working electrode assembly is a patch-type structure.

The term "patch" conventionally refers to a cloth-like piece having adhesiveness in one surface mainly containing a substance such as a drug or an antigen. The structure of the patch may have some degree of thickness, and the patch has no adhesiveness in some cases.

As described in, for example, JP 2002-532540 A, a nicotine patch containing nicotine as an antismoking adjuvant has been known as an example of such patch. In addition, as described in JP 2005-510488 A, some transdermal absorption patches are used as local anesthetic drugs using morphine hydrochloride and the like.

In such iontophoresis device as described above, a current supplied from a DC electric power source must be adjusted depending on the conditions of an organism to which a patch-type working electrode assembly or non-working electrode assembly is stuck such as the impedance of the skin of the organism.

In addition, the amount of the current is desirably adjusted in such a manner that a patient cannot change the amount once a doctor has set the amount.

On the other hand, conventional current amount adjusting means or current amount setting means has so large a volume that it cannot be provided for such patch-type iontophoresis device as described above.

### Disclosure of the Invention

An obj ect of the present invention is to constitute a DC electric power source in a patch-type iontophoresis device in such a manner that the DC electric power source has a small size and its current amount cannot be changed once the amount has been set by a doctor.

The above object can be achieved by embodiments as described below.

(1) A patch-type iontophoresis device including: a working electrode assembly and a non-working electrode assembly each used for administering an ionic drug by means of iontophoresis; and a DC electric power source connected to a working electrode of the working electrode assembly and a non-working electrode of the non-working electrode assembly with opposite polarities,
characterized in that: the device further includes an electrically conductive circuit including a film-like electrically conductive cord provided between the DC electric power source and at least one of the working electrode of the working electrode assembly and the non-working electrode of the non-working electrode assembly; and a current value setting device capable of irreversibly setting a value of current from the DC electric power source by means of one of switching and partial blocking of the electrically conductive circuit.

(2) The patch-type iontophoresis device according to the above item (1), characterized in that: the current value setting device includes a film-like electrical resistor and the electrically conductive cord arranged in parallel with each other and a film-like connector stuck so as to selectively connect an arbitrary portion in a longitudinal direction of the electrical resistor and an arbitrary portion in a longitudinal direction of the electrically conductive cord; one end in the longitudinal direction of the electrical resistor is electrically blocked; and the end of the electrically conductive cord on an opposite side to the one end of the electrical resistor in the longitudinal direction is electrically blocked.

(3) The patch-type iontophoresis device according to the above item (1), characterized in that: a part or entirety of the electrically conductive cord in the electrically conductive circuit in a direction of electrical conductivity is a cut portion; at least two kinds of electrically conductive cords for setting capable of being stuck to the cut portion and having different resistance values or different current amounts are prepared; and the electrically conductive cords are capable of being selectively stuck as a part of the electrically conductive circuit from the working electrode to the non-working electrode.

(4) The patch-type iontophoresis device according to the above item (3), characterized in that the electrically conductive cords for setting each include a film composed of an insulating material, a metal film formed on one surface of the film, and an adhesive layer provided on a periphery of the metal film on the one surface of the film.

(5) The patch-type iontophoresis device according to the above item (4), characterized in that the at least two kinds of the electrically conductive cords for setting are formed on one surface of the same film with an adhesive layer provided on a periphery of each of the electrically conductive cords.

(6) The patch-type iontophoresis device according to the above item (1), characterized in that the current value setting device includes at least two kinds of electrically conductive circuits having different resistance values or different current amounts, the electrically conductive circuits being arranged in parallel with each other, and a selective setting device capable of blocking conduction of the electrically conductive cord except for at least one of the at least two kinds of the electrically conductive circuits.

(7) The patch-type iontophoresis device according to the above item (6), characterized in that the electrically conductive cord is composed of a metal film, and the selective setting device has a pull member capable of removing a part of the metal film in a direction of electric current.

(8) The patch-type iontophoresis device according to the above item (6), characterized in that the electrically conductive circuits include constant current diodes, respectively, having control current values different from each other.

(9) The patch-type iontophoresis device according to any one of the above items (6) to (8), characterized in that: two kinds of the electrically conductive circuits are set; and the selective setting device is capable of selecting one of a mode for blocking conduction of one of the two kinds of the electrically conductive circuits and a mode for blocking conduction of none of the two kinds of the electrically conductive circuits.

(10) The patch-type iontophoresis device according to any one of the above items (1) to (9), characterized in that: a tape-like connecting portion for coupling and electrically connecting the working electrode assembly with the non-working electrode assembly is provided; and the current value setting device is provided in the tape-like connecting portion.

(11) The patch-type iontophoresis device according to any one of the above items (1) to (10), characterized in that: the working electrode assembly includes: the working electrode connected to the DC electric power source with the same polarity as that of a charged ion of the ionic drug; an electrolyte solutionholdingportion holding an electrolyte solution, the electrolyte solution holding portion being placed on a front surface of the working electrode; a second ion exchange membrane selecting an ion of an opposite polarity to that of the charged ion of the ionic drug, the second ion exchange membrane being placed on a front surface of the electrolyte solution holding portion; a drug solution holding portion holding the ionic drug, the drug solution holding portion being placed on a front surface of the second ion exchange membrane; and a first ion exchange membrane selecting an ion of the same polarity as that of the charged ion of the ionic drug, the first ion exchange membrane being placed on a front surface of the drug solution holding portion; and the non-working electrode assembly includes: the non-working electrode connected to the DC electric power source with the opposite polarity to that of the charged ion of the ionic drug; a second electrolyte solution holding portion holding a second electrolyte solution, the second electrolyte solution holding portion being placed on a front surface of the non-working electrode; a third ion exchange membrane selecting an ion of the opposite polarity to that of the charged ion of the ionic drug, the third ion exchange membrane being placed on a front surface of the second electrolyte solution holding portion; a third electrolyte solution holding portion holding a third electrolyte solution, the third electrolyte solution holding portion being placed on a front surface of the third ion exchange membrane; and a fourth ion exchange membrane selecting an ion of the opposite polarity to that of the charged ion of the ionic drug, the fourth ion exchange membrane being placed on a front surface of the third electrolyte solution holding portion.

### Brief Description of the Drawings

[Fig. 1] A plan view showing a patch-type iontophoresis device according to a first embodiment of the present invention.
[Fig. 2] An enlarged sectional view taken along the line II-II of Fig. 1.
[Fig. 3] An enlarged sectional view taken along the line III-II of Fig. 1.
[Fig. 4] A circuit diagram showing an electrical system in the patch-type iontophoresis device.
[Fig. 5] An enlarged sectional view showing a part of a current value setting device in the patch-type iontophoresis device.
[Fig. 6] A perspective view showing a main portion of a patch-type iontophoresis device according to a second embodiment of the present invention.
[Fig. 7] An enlarged perspective view showing a part of the current value setting device.
[Fig. 8] A perspective view showing a main portion of a patch-type iontophoresis device according to a third embodiment of the present invention.
[Fig. 9] An enlarged perspective view showing a part of the current value setting device.
[Fig. 10] A plan view showing a main portion of a patch-type iontophoresis device according to a fourth embodiment of the present invention.

### Best Mode for carrying out the Invention

Hereinafter, the best mode for carrying out the present invention will be described with reference to the drawings.

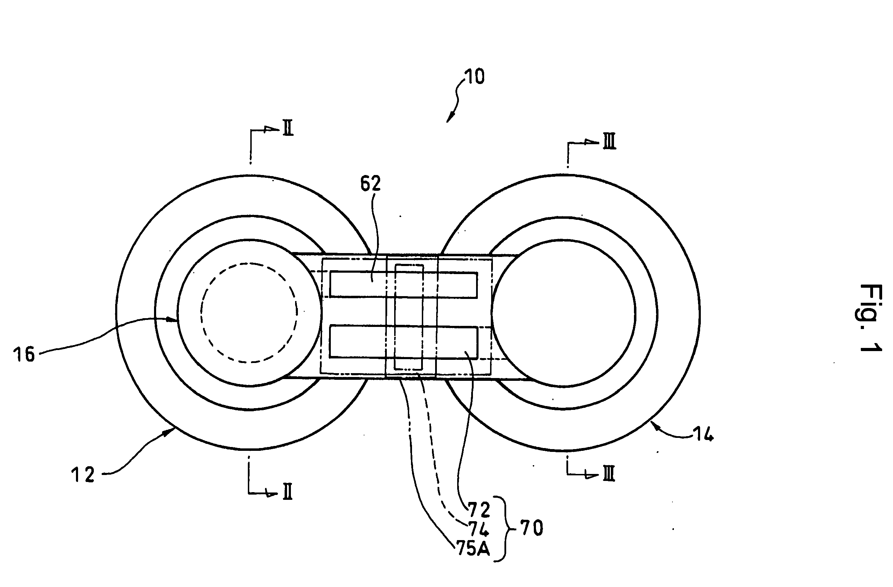

As shown in Fig. 1, a patch-type iontophoresis device 10 according to the best mode is constituted by a working electrode assembly 12 and a non-working electrode assembly 14 each used for administering an ionic drug, a DC electric power source 16 connected to the electrode assemblies 12 and 14 with opposite polarities, an electrically conductive circuit 60 including an electrically conductive cord 62 for electrically connecting the DC electric power source 16 and the non-working electrode assembly 14, and a current value setting device 70 capable of irreversibly setting a value of a current from the DC electric power source 16 flowing through the electrically conductive circuit 60.

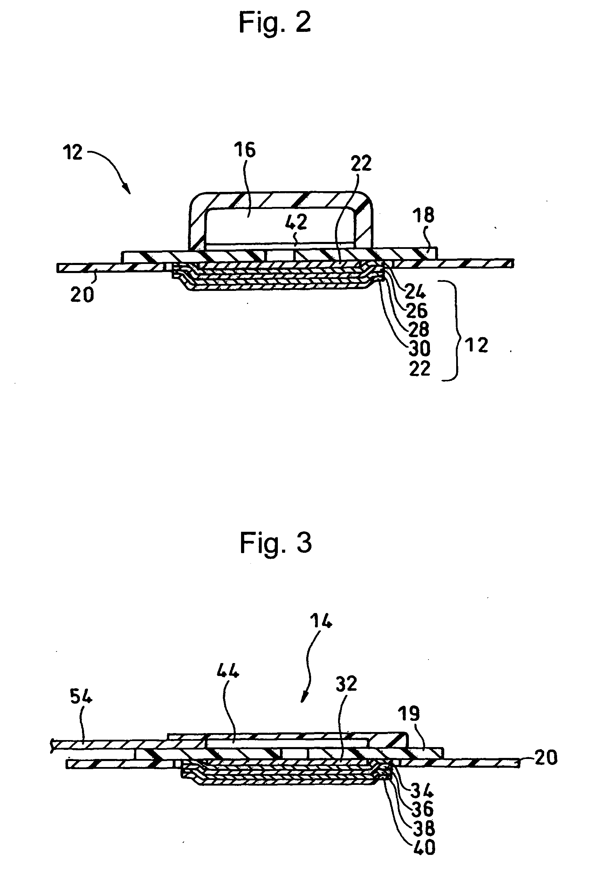

Fig. 2 shows the section of the working electrode assembly 12. The working electrode assembly 12 is constituted by laminating a working electrode 22, an electrolyte solution holding portion 24, a second ion exchange membrane 26, a drug solution holding portion 28, and a first ion exchange membrane 30 in this order from the lower side in Fig. 2 and its plane shape is a circle of 10 to 40 mm in diameter. A toric adhesive sheet 20 is arranged on the lower surface in Fig. 2 of the base sheet 18 so as to surround the working electrode 22 to the first ion exchange membrane 30.

The base sheet 18 is preferably constituted by a resin material which is hard, insulative, and elastic such as a PET (polyethylene terephthalate) resin, and is adapted to press the working electrode 22 to the first ion exchange membrane 30 against the skin or mucosa of an organism when the sheet 18 is pulled with the adhesive sheet 20.

The working electrode 22 is desirably constituted by a conductive coating applied to the one surface of the base sheet 18 and blended with a nonmetal conductive filler such as a carbon paste. The working electrode 22 can be constituted by a copper plate or a metal thin film, but a metal eluted from the plate or the thin film may transfer to an organism upon administration of a drug. Therefore, the working electrode 22 is preferably nonmetallic.

The electrolyte solution holding portion 24 is constituted by, for example, an electrolytic coating applied to the working electrode 22. The electrolytic coating is a coating containing an electrolyte, and examples of an electrolyte particularly preferably used include: medical agents such as ascorbic acid (vitamin C) and sodium ascorbate; and organic acids such as lactic acid, oxalic acid, malic acid, succinic acid, and fumaric acid and/or salts thereof. The use of such electrolyte can suppress the generation of an oxygen gas or a hydrogen gas. In addition, blending a plurality of kinds of electrolytes serving as a combination of buffer electrolyte solutions upon dissolution in a solvent can suppress a change in pH during energization.

The electrolytic coating is blended with a hydrophilic polymer such as polyvinyl alcohol, polyacrylic acid, polyacrylamide, or polyethylene glycol in order to improve the application property and film-forming property of the coating, and is blended with an appropriate amount of solvent such as water, ethanol, or propanol for adjusting the viscosity of the electrolytic coating. The coating may be blended with an appropriate additional component such as a thickener, a thixotropic agent, a defoaming agent, a pigment, a flavor, or a coloring agent.

The second ion exchange membrane 26 is formed by applying a second ion exchange coating to the electrolyte solution holding portion 24.

The second ion exchange coating is a coating containing an ion exchange resin into which an ion exchange group using an ion having a conductivity type opposite to that of a drug ion in the drug solution holding portion 28 to be described later as a counter ion is introduced. In the case where a drug whose drug component dissociates to positive drug ions is used in the drug solution holding portion 28, the coating is blended with an anion exchange resin. On the other hand, in the case where a drug whose drug component dissociates to negative drug ions is used, the coating is blended with a cation exchange resin.

The drug solution holding portion 28 is composed of a drug coating applied to the second ion exchange membrane 26. The coating is a coating containing a drug (including a precursor for the drug) whose drug component dissociates to positive or negative ions (drug ions) as a result of, for example, dissolution into a solvent such as water. Examples of a drug whose drug component dissociates to positive ions include lidocaine hydrochloride as an anesthetic drug and morphine hydrochloride as an anesthetic drug. Examples of a drug whose drug component dissociates to negative ions include ascorbic acid as a vitamin agent.

The first ion exchange membrane 30 is formed of a first ion exchange coating applied to the drug solution holding portion 28. The first ion exchange coating is a coating containing an ion exchange resin into which an ion exchange group using an ion having the same conductivity type as that of the drug ion in the drug solution holding portion 28 as a counter ion is introduced. In the case where a drug whose drug component dissociates to positive/negative drug ions is used in the drug solution holding portion 28, the coating is blended with an anion/cation exchange resin.

An ion exchange resin obtained by introducing a cation exchange group (an exchange group using a cation as a counter ion) such as a sulfonic group, a carboxylic group, or a phosphoric group into a polymer having a three-dimensional network structure such as a hydrocarbon-based resin (for example, a polystyrene resin or an acrylic resin) or a fluorine-based resin having a perfluorocarbon skeleton can be used as the cation exchange resin without any limitation.

An ion exchange resin obtained by introducing an anion exchange group (an exchange group using an anion as a counter ion) such as a primary amino group, a secondary amino group, a tertiary amino group, a quaternary ammonium group, a pyridyl group, an imidazole group, a quaternary pyridinium group, or a quaternary imidazolium group into a polymer having a three-dimensional network structure similar to that in the case of the cation exchange resin can be used as the anion exchange resin without any limitation.

Fig. 3 is an enlarged view showing that the non-working electrode assembly 14 is constituted by laminating a non-working electrode 32, a second electrolyte solution holding portion 34, a third ion exchange membrane 36, a third electrolyte solution holding portion 38, and a fourth ion exchange membrane 40 arranged on the lower side in the figure of a non-working base sheet 19 similar to the base sheet 18 in this order and its plane shape is the same as that of the working electrode assembly 12.

The non-working electrode 32 has the same constitution as that of the working electrode 22 in the working electrode assembly 12, and the constitutions and components of the second electrolyte solution holding portion 34 and the third electrolyte solution holding portion 38 are the same as those of the electrolyte solution holding portion 24.

The third ion exchange membrane 36 is formed of an ion exchange coating applied to the second electrolyte solution holding portion 34. The ion exchange coating is the same as the first ion exchange coating of which the first ion exchange membrane 30 is formed, and the third ion exchange membrane 36 functions as an ion exchange membrane similar to the first ion exchange membrane 30.

The fourth ion exchange membrane 40 is formed of the same second ion exchange coating as that described above applied to the third electrolyte solution holding portion 38. The fourth ion exchange membrane 40 functions as an ion exchange membrane similar to the second ion exchange membrane 26.

Aworking electrode terminal 42 is arranged on the upper surface in Fig. 2 of the base sheet 18 in the working electrode assembly 12, and conduction is established between the working electrode terminal 42 and the working electrode 22 of the working electrode assembly 12 through a through-hole arranged on the base sheet 18.

Similarly, a non-working electrode terminal 44 is arranged on the upper surface in Fig. 3 of the non-working base sheet 19 in the non-working electrode assembly 14, and conduction is established between the non-working electrode terminal 44 and the non-working electrode 32 of the non-working electrode assembly 14 through a through-hole formed on the non-working base sheet 19.

The DC electric power source 16 is arranged to cover the upper side of the working electrode terminal 42.

The DC electric power source 16 is constituted by a button battery 46 and a mold resin 50 composed of an insulating material for integrally molding the button battery 46.

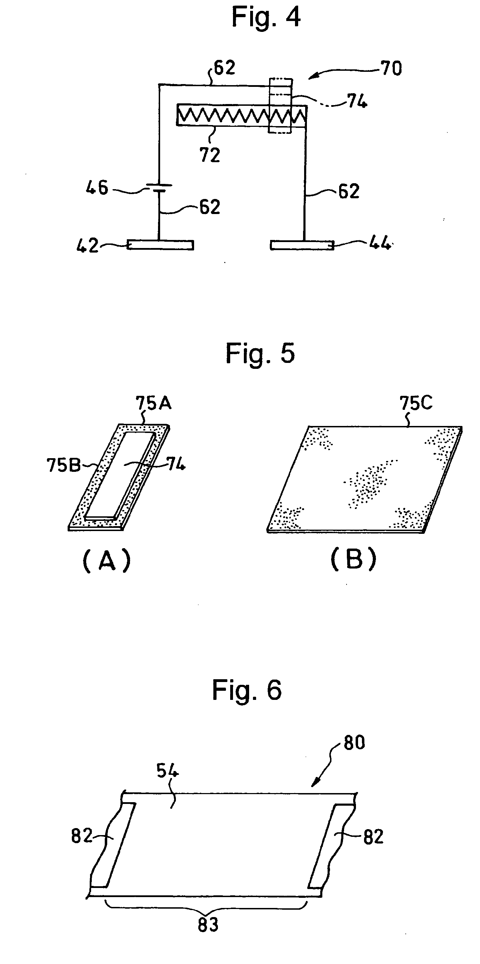

As shown in Fig. 4 (a circuit diagram), the cathode side of the button battery 46 in the DC electric power source 16 is connected to the working electrode terminal 42, and the cathode side thereof is connected to the non-working electrode terminal 44 on the side of the non-working electrode assembly 14 via the electrically conductive cord 62.

Reference numeral 54 in Fig. 1 denotes a coupling belt composed of, for example, a PET film for coupling the base sheet 18 and the non-working base sheet 19. The electrically conductive cord 62 is arranged along the surface of the coupling belt 54.

The electrically conductive cord 62 is composed of a metal film formed on the coupling belt 54, and the current value setting device 70 includes a film-like electrical resistor 72 arranged in parallel with the electrically conductive cord 62 and a connector 74 which is composed of a metal film and can be stuck at an arbitrary portion between the electrically conductive cord 62 and the electrical resistor 72 arranged in parallel so as to selectively connect the electrically conductive cord 62 and the electrical resistor 72 upon sticking.

Here, the electrically conductive cord 62 is electrically blocked by being cut at an end on the side of the non-working electrode assembly 14. The end of the resistor 72 on the side of the working electrode assembly 12 is similarly electrically blocked, and the end thereof on the opposite side is connected to the electrically conductive cord 62 on the side of the non-working electrode 44.

As shown in Fig. 5, the connector 74 is composed of a metal film formed on one surface of an insulating film 75 A. An adhesive layer 75B is arranged at a part of the insulating film 75A surrounding the connector 74. When the connector 74 is attached to the coupling belt 54 so as to cross the electrically conductive cord 62 and the electrical resistor 72 at an arbitrary portion in the longitudinal direction of each of the cord and the resistor, the adhesive layer 75B is unpeelably stuck in a state where conduction is established between the electrically conductive cord 62 and the electrical resistor 72 by the connector 74. Furthermore, an insulating cover film 75C having an adhesive layer is stuck to cover exposed portions of the electrically conductive cord 62 and the electrical resistor 72 so that the adhesive layer cannot be peeled.

The resistance value of a newly formed electrically conductive circuit is set by the position of conduction between the electrically conductive cord 62 and the electrical resistor 72 formed by the connector 74, that is, the length of the electrical resistor 72 in which a current from the DC electric power source 16 flows. Accordingly, the value of a current flowing in the electrically conductive circuit 60 is irreversibly set.

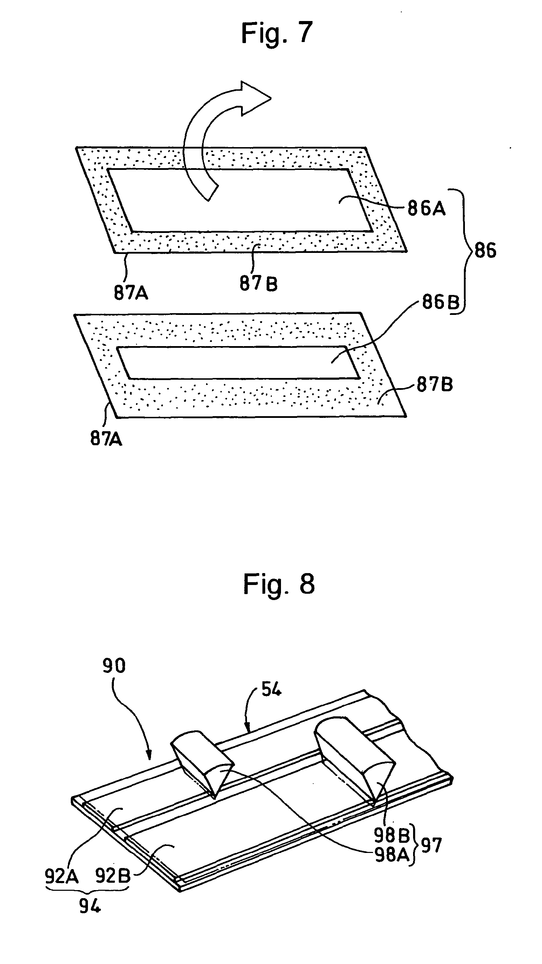

Next, a patch-type iontophoresis device 80 according to a second embodiment of the present invention will be described with reference to Figs. 6 and 7.

The patch-type iontophoresis device 80 (the entirety of which is not shown) includes an electrically conductive cord 82, an electrically conductive circuit 84, and a current value setting device 86 having constitutions different from those of the electrically conductive circuit 60 including the electrically conductive cord 62 and the current value setting device 70. The other constitutions are the same as those of the patch-type iontophoresis device 10. Accordingly, the same reference symbols as those of the patch-type iontophoresis device 10 are given to the same constituent parts, and description of the parts is omitted.

The range of the electrically conductive cord 82 in the patch-type iontophoresis device 80 according to the second embodiment in its direction of electrical conductivity having substantially the same length as that of the coupling belt 54 is a cut portion 83. The current value setting device 86 is capable of being stuck to the cut portion 83, and is constituted by at least two kinds of electrically conductive cords 86A and 86B for setting having different resistance values or different current amounts. Upon setting of the value of a current, one of the electrically conductive cords 86A and 86B for setting is chosen in accordance with the value of a current to be set, and is stuck as a part of the electrically conductive cord 82 to compensate for the cut portion 83.

The electrically conductive cords 86A and 86B for setting are obtained by forming an electrically resistive material into a film-like shape on the surface of an insulating film 87A, and the periphery of each of the cords is surrounded by an adhesive layer 87B.

Accordingly, in the case of the current value setting device 86, the electrically conductive cord 86A or 86B for setting is chosen in accordance with the value of a current to be set, and the selected cord is stuck to the coupling belt 54 with the aid of the adhesive layer 87B in such a manner that the selected cord fills the cut portion 83 of the electrically conductive cord 82 to bring the electrically conductive cord 82 into an electrically conductive state.

It should be noted that the electrically conductive cords 86A and 86B for setting may be formed on one surface of one insulating film in a state of being surrounded by an adhesive layer and may be separated from each other before use.

Next, a patch-type iontophoresis device 90 (the entirety of which is not shown) according to a third embodiment of the present invention shown in Fig. 8 will be described.

The third embodiment employs an electrically conductive circuit 94 including electrically conductive cords 92A and 92B, and a current value setting device 96 having constitutions different from those of the first and second embodiments. The other constitutions are the same as those of the first and second embodiments. Accordingly, the same reference symbols as those of the first and second embodiments are given to the same constitutions, and description of the constitutions is omitted.

The current value setting device 96 in the patch-type iontophoresis device 90 is constituted by the at least two kinds of electrically conductive cords 92A and 92B arranged in parallel and having different resistance values or different current amounts. The current value setting device 96 constituted by the electrically conductive circuits 92A and 92B includes a selective setting device 97 capable of blocking conduction of one of the electrically conductive circuits 92A and 92B.

Specifically, each of the electrically conductive cords 92A and 92B is composed of a metal film, and the selective setting device 97 includes blocking buttons 98A and 98B capable of breaking or cutting the electrically conductive cords 92A and 92B by pressing in the thickness directions of the cords.

Each of the blocking buttons 98A and 98B is composed of an insulating material such as a resin attached to a hole portion of a cover film 99 with which the electrically conductive cords 92A and 92B are covered. The tips of the buttons are each formed to have an acute angle so that they can cut or break the film-like electrically conductive cords 92A and 92B when the cover film 99 is pressed in its thickness direction. After that, the buttons can be removed and wasted.

Accordingly, the selective setting device 97 is capable of setting the value of a current flowing in the electrically conductive circuit 94 in three stages on the basis of three modes: a mode for blocking conduction of the electrically conductive cord 92A, a mode for blocking conduction of the electrically conductive cord 92B, and a mode for blocking conduction of none of the electrically conductive cords 92A and 92B. After the value has been set, the blocking buttons 98A and 98B are removed, and their marks are covered with an adhesive tape or the like. In this case, the setting cannot be changed thereafter.

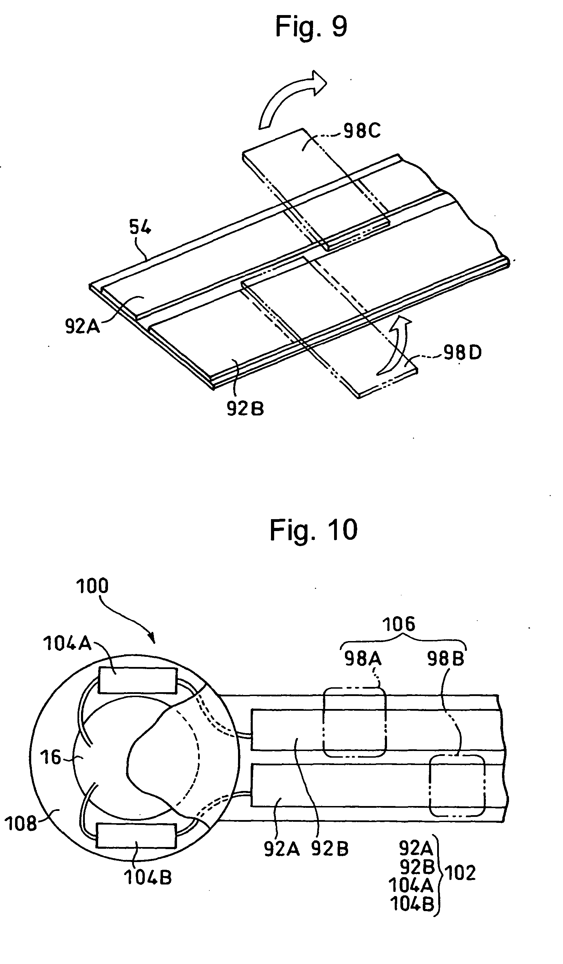

The selective setting device 97 selectively cuts or breaks the electrically conductive cords 92A and 92B with the aid of the blocking buttons 98A and 98B, or blocks conduction of none of the cords. Alternatively, a current value can be set in three stages as follows. As shown in Fig. 9, parts of the electrically conductive cords 92A and 92B in their directions of electrical conductivity are designed to be removal instead of placing the blocking buttons 98A and 98B, and pull members 98C and 98D each composed of a film or the like are stuck in advance to the parts. Upon setting of a current value, one of the pull members 98C and 98D is pulled from the outside to block the electrically conductive cord 92A or 92B or to block none of the electrically conductive cords 92A and 92B.

A current value can be prevented from being changed by a user as long as ends of the pull members 98C and 98D projecting toward the outside are separated from each other after the current value has been set.

Next, a patch-type iontophoresis device 100 (the entirety of which is not shown) according to a fourth embodiment of the present invention shown in Fig. 10 will be described.

The patch-type iontophoresis device 100 is different from those of the first to third embodiments in the constitution of the DC electric power source 16 and the constitutions of an electrically conductive circuit including an electrically conductive cord and of a current value setting device.

An electrically conductive circuit 102 in the patch-type iontophoresis device 100 includes constant current diodes 104A and 104B having control current values different from each other. A current value setting device 106 is adapted to operate in a mode for selectively blocking connection of one of the constant current diodes 104A and 104B to the DC electric power source 16 or a mode for blocking connection of none of the diodes to the power source.

Specifically, it is recommended that the blocking buttons 98A and 98B or the pull members 98C and 98D for selectively blocking conduction of one of the electrically conductive cords 92A and 92B or for blocking conduction of none of the cords be used for the current value setting device 106.

As shown in Fig. 10, the constant current diodes 104A and 104B are arranged laterally to or on the DC electric power source 16 composed of a button cell, and are integrally molded into a flat shape by a mold resin 108.

In the patch-type iontophoresis device 100, a current flows through the two constant current diodes 104A and 104B to have a maximum value in the case where a mode for blocking conduction of none of the constant current diodes 104A and 104B is selected upon setting of a current value.

In each of the patch-type iontophoresis devices 10, 80, 90, and 100, each of the working electrode assembly 12 and the non-working electrode assembly 14 has a pair of ion exchange membranes. However, the present invention is not limited thereto. The present invention is applicable also to the case where each of them has one ion exchange membrane.

Each of the current value setting devices 70, 86, 96, and 106 is arranged at a position on the coupling belt 54 in each of the embodiments. However, the present invention is not limited thereto. Each of the devices may be arranged in the working electrode assembly 12 or the non-working electrode assembly 14, or may be arranged across the assemblies and the coupling belt.

### Industrial Applicability

In the patch-type iontophoresis device of present invention, the amount of a current supplied from a small-sized DC electric power source to a working electrode or a non-working electrode can be irreversibly set by a method for switching or partial blocking of an electrically conductive circuit including a film-like electrically conductive structure. Then, the doctor may have expectation of his patient to use the patch-type iontophoresis device reasonably.

## Claims

1. A patch-type iontophoresis device comprising:
a working electrode assembly and a non-working electrode assembly each used for administering an ionic drug by means of iontophoresis; and
a DC electric power source connected to a working electrode of the working electrode assembly and a non-working electrode of the non-working electrode assembly with opposite polarities,
**characterized in that**:
the device further comprises an electrically conductive circuit including a film-like electrically conductive cord provided between the DC electric power source and at least one of the working electrode of the working electrode assembly and the non-working electrode of the non-working electrode assembly; and
a current value setting device capable of irreversibly setting a value of current from the DC electric power source by means of one of switching and partial blocking of the electrically conductive circuit.

2. The patch-type iontophoresis device according to claim 1, **characterized in that**:
the current value setting device comprises
a film-like electrical resistor and the electrically conductive cord arranged in parallel with each other and
a film-like connector stuck so as to selectively connect an arbitrary portion in a longitudinal direction of the electrical resistor and an arbitrary portion in a longitudinal direction of the electrically conductive cord;
one end in the longitudinal direction of the electrical resistor is electrically blocked; and
an end of the electrically conductive cord on an opposite side to the one end of the electrical resistor in the longitudinal direction is electrically blocked.

3. The patch-type iontophoresis device according to claim 1, **characterized in that**:
a part or entirety of the electrically conductive cord in the electrically conductive circuit in a direction of electrical conductivity is a cut portion;
at least two kinds of electrically conductive cords for setting capable of being stuck to the cut portion and having different resistance values or different current amounts are prepared; and
the electrically conductive cords are capable of being selectively stuck as a part of the electrically conductive circuit from the working electrode to the non-working electrode.

4. The patch-type iontophoresis device according to claim 3, **characterized in that** the electrically conductive cords for setting each comprise
a film comprising an insulating material,
a metal film formed on one surface of the film, and
an adhesive layer provided on a periphery of the metal film on the one surface of the film.

5. The patch-type iontophoresis device according to claim 4, **characterized in that** the at least two kinds of the electrically conductive cords for setting are formed on one surface of the same film with an adhesive layer provided on a periphery of each of the electrically conductive cords.

6. The patch-type iontophoresis device according to claim 1, **characterized in that** the current value setting device comprises
at least two kinds of electrically conductive circuits having different resistance values or different current amounts, the electrically conductive circuits being arranged in parallel with each other and
a selective setting device capable of blocking conduction of the electrically conductive cord except for at least one of the at least two kinds of the electrically conductive circuits.

7. The patch-type iontophoresis device according to claim 6, **characterized in that**
the electrically conductive cord comprises a metal film; and
the selective setting device comprises a pull member capable of removing a part of the metal film in a direction of electric current.

8. The patch-type iontophoresis device according to claim 6, **characterized in that** the electrically conductive circuits comprise constant current diodes, respectively, having control current values different from each other.

9. The patch-type iontophoresis device according to any one of claims 6 to 8, **characterized in that**:
two kinds of the electrically conductive circuits are set; and
the selective setting device is capable of selecting one of a mode for blocking conduction of one of the two kinds of the electrically conductive circuits and a mode for blocking conduction of none of the two kinds of the electrically conductive circuits.

10. The patch-type iontophoresis device according to any one of claims 1 to 9, **characterized in that**:
a tape-like connecting portion for coupling and electrically connecting the working electrode assembly with the non-working electrode assembly is provided; and
the current value setting device is provided in the tape-like connecting portion.

11. The patch-type iontophoresis device according to any one of claims 1 to 10, **characterized in that**:
the working electrode assembly comprises:
the working electrode connected to the DC electric power source with the same polarity as that of a charged ion of the ionic drug;
an electrolyte solution holding portion holding an electrolyte solution, the electrolyte solution holding portion being placed on a front surface of the working electrode for holding an electrolyte solution;
a second ion exchange membrane selecting an ion of an opposite polarity to that of the charged ion of the ionic drug, the second ion exchange membrane being placed on a front surface of the electrolyte solution holding portion;
a drug solution holding portion holding the ionic drug, the drug solution holding portion being placed on a front surface of the second ion exchange membrane; and
a first ion exchange membrane selecting an ion of the same polarity as that of the charged ion of the ionic drug, the first ion exchange membrane being placed on a front surface of the drug solution holding portion; and
the non-working electrode assembly comprises:
the non-working electrode connected to the DC electric power source with the opposite polarity to that of the charged ion of the ionic drug;
a second electrolyte solution holding portion holding a second electrolyte solution, the second electrolyte solution holding portion being placed on a front surface of the non-working electrode;
a third ion exchange membrane selecting an ion of the opposite polarity to that of the charged ion of the ionic drug, the third ion exchange membrane being placed on a front surface of the second electrolyte solution holding portion;
a third electrolyte solution holding portion holding a third electrolyte solution, the third electrolyte solution holding portion being placed on a front surface of the third ion exchange membrane; and
a fourth ion exchange membrane selecting an ion of the opposite polarity to that of the charged ion of the ionic drug, the fourth ion exchange membrane being placed on a front surface of the third electrolyte solution holding portion.
